# FASCICULE DE BREVET EUROPEEN

(11) **EP 0 525 493 B1**
(45) Date de publication et mention de la délivrance du brevet: **06.09.1995**
(21) Numéro de dépôt: 92111904.6
(22) Date de dépôt: 13.07.1992
(51) Int. Cl.: A61M 1/00

(54) **Appareil de récupération et de filtration du sang**
Blutsammlungs- und Filtrationsvorrichtung
Blood recuperating and filtration device

(30) Priorité: 26.07.1991 CH 2249/91
(43) Date de publication de la demande: 03.02.1993
(73) Titulaire: ELP ROCHAT, CH-1295 Mies (CH)
(72) Inventeur: Rochat, Jean-Denis, CH-1295 Mies (CH)
(74) Mandataire: Micheli & Cie

(56) Documents cités:
- EP-A- 0 040 427
- WO-A-88/10124
- DE-A- 1 810 801
- DE-A- 3 328 562
- US-A- 4 346 711

## Description

La présente invention concerne un appareil pour la récupération et la filtration stérile du sang per- et post-opératoire, ainsi qu'une poche souple de filtration jetable pour cet appareil.

Ce type d'appareil fonctionnant par aspiration sous vide est destiné à être utilisé pour une filtration grossière du sang, avant que celui-ci soit conduit dans un appareil de lavage du sang et le cas échéant réinfusé dans le même patient (auto-transfusion).

On connaît déjà de tels appareils, par exemple du brevet US 4 466 888, dans lesquels une poche de récupération du sang est fixée par pincement le long de ses bords périphériques entre les deux parties d'une coquille rigide. Toutefois, de tels appareils se sont révélés pratiquement inutilisables pour des collectes de sang post-opératoires, étant donné que l'étanchéité du système est insuffisante une fois la ligne de vide débranchée. Il existe d'autres types de tels appareils, qui présentent tous des inconvénients, soit à cause de la complexité de leur utilisation ou de leur fabrication, soit du fait d'un coût trop élevé, soit encore parce qu'ils sont tout simplement inefficaces.

Le but de la présente invention consiste donc à fournir un appareil pour la récupération et la filtration stérile du sang per- et post-opératoire qui permette de remédier aux inconvénients des appareils connus, et qui soit par conséquent simple à fabriquer et à utiliser, mais néanmoins efficace, et dont la partie jetable surtout soit peu coûteuse.

Le but précité est obtenu avec l'appareil selon l'invention qui présente les caractéristiques définies dans la revendication 1.

Un autre objet de l'invention consiste en une poche souple stérile et jetable pour la récupération et la filtration du sang per- et post-opératoire, utilisable dans l'appareil selon l'invention, et qui est tel que défini dans la revendication 3.

Le dessin annexé illustre schématiquement et à titre d'exemple une forme d'exécution de l'appareil et de la poche souple jetable selon l'invention.

La figure 1 en est une vue générale en position de service et partiellement en coupe.

La figure 2 est une vue en plan de la poche souple jetable en position hors service, et la figure 3 est une vue en coupe d'un détail de cette poche.

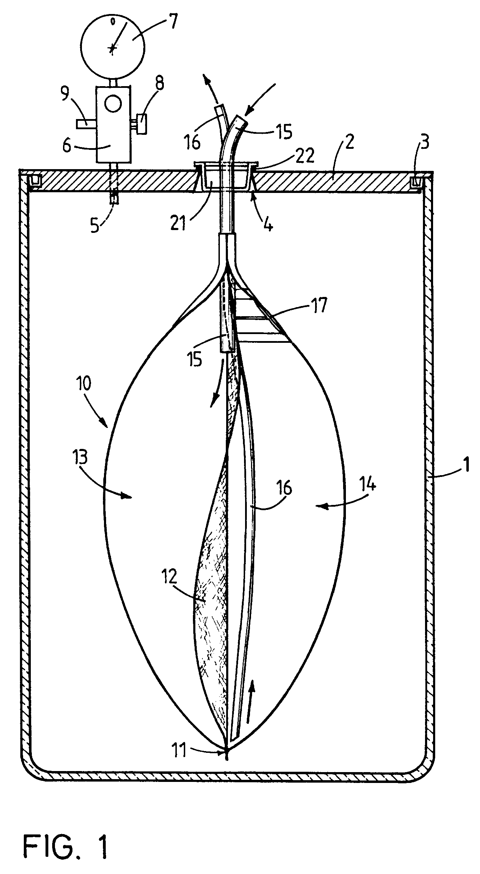

En référence tout d'abord à la figure 1, l'appareil selon l'invention comprend un récipient cylindrique 1 de préférence en verre ou en matière plastique rigide dont l'ouverture supérieure peut être fermée hermétiquement par un couvercle rigide 2, de préférence en métal, l'étanchéité étant assurée par un joint par exemple un joint silicone en V. Le couvercle 2 présente une ouverture centrale 4 et un orifice d'entrée du vide 5. Sur ce dernier orifice est monté fixe un dispositif régulateur de pression 6, muni d'un manomètre de contrôle 7, d'une molette de réglage 8 et d'un tube de branchement 9. L'ensemble récipient 1-couvercle 2-dispositif régulateur de vide 6 constitue la partie fixe non jetable de l'appareil selon l'invention.

Quant à la partie jetable de l'appareil selon l'invention, telle qu'elle est illustrée à titre d'exemple sur les figures 1 à 3, elle comprend une poche souple 10, de préférence en PVC, formée par deux parois assemblées à plat par leur rebords périphériques 11. Vu en plan, cette poche 10 présente une forme conique à ses extrémités supérieure et inférieure, de manière à faciliter son introduction dans respectivement son retrait du récipient 1 à travers l'ouverture centrale 4 du couvercle 2.

Lors de la fabrication de cette poche 10, une paroi interne de filtration 12, dont la forme à plat est identique à celle des parois de la poche 10, est soudée également par sa périphérie entre les deux rebords 11 de ces deux parois. Cette paroi de filtration est de préférence formée par une grille en "Nylon" ayant des mailles entre 100 et 1000 microns, de préférence de l'ordre de 300 microns, et divise l'intérieur de la poche 10 en deux chambres 13,14 d'égal volume. Chacune de ces chambres 13,14 est reliée à l'extérieur de la poche 10 par un tuyau souple 15,16 et chaque tuyau étant fixé de manière étanche par soudage avec les rebords 11 des deux parois de la poche.

La première chambre 13, dans laquelle débouche le tuyau 15, est complètement étanche et est destinée à recevoir le sang souillé à filtrer, par exemple après opération, c'est-à-dire qui contient des caillots, des débris d'os, etc. Le sang est aspiré dans cette première chambre 13 grâce à la mise sous pression réduite (ci-après dénommée "vide") de l'appareil selon l'invention.

La seconde chambre 14 présente en effet une ouverture, par exemple de forme rectangulaire allongée, munie d'une bande 17 en un matériau hydrophobe, c'est-à-dire qui est imperméable aux liquides mais perméable à l'air, et qui permet de mettre cette seconde chambre sous la même pression que l'intérieur du cylindre 1. Le tuyau 16 introduit dans cette seconde chambre 14 va jusqu'au fond de celle-ci, ce tuyau étant destiné à pomper le sang filtré dans cette chambre 14 pour l'amener ensuite par exemple vers un appareil de lavage du sang, avant d'être le cas échéant réintroduit dans le système sanguin du même patient (auto-transfusion).

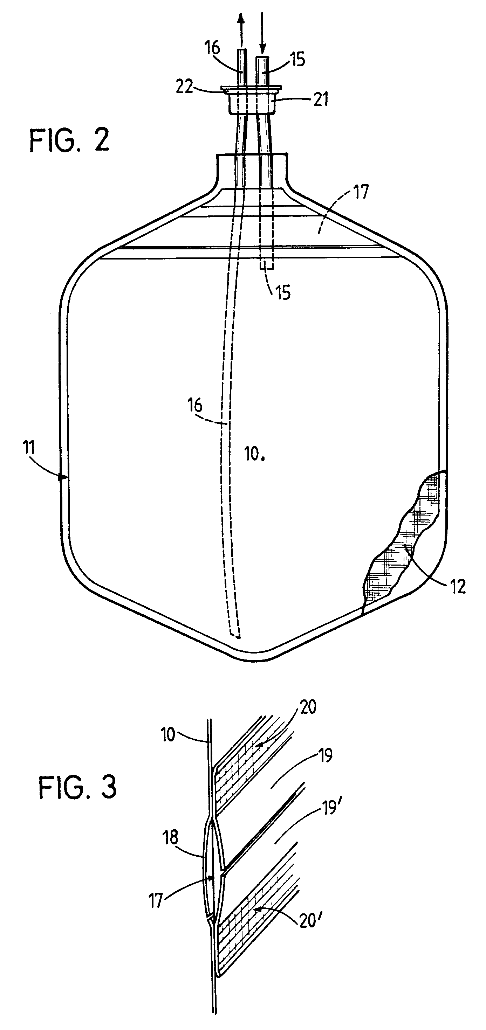

Pour des raisons de fabrication essentiellement, et comme illustré sur la figure 3, la bande hydrophobe 17, par exemple en un matériau du type "Filtre Millipore", est fixée entre une portion 18 de la paroi extérieure de la poche 10, dans laquelle une fente de passage horizontale a été découpée, et deux rabats 19,19' en une matière plastique identique à celle de la paroi 18 (par exemple du PVC), au moyen de deux soudures horizontales 20,20'.

Enfin, les deux tuyaux souples 15,16, respectivement pour l'entrée et la sortie du sang de la poche 10, sont soudés à un bouchon 21 destiné à être introduit dans l'ouverture centrale 4 du couvercle 2, ceci de manière étanche grâce à un joint circulaire 22.

Pour l'introduction de la poche souple jetable 10 dans le récipient 1, il convient d'abord d'enrouler cette poche autour de son axe vertical, puis de l'enfiler sous cette forme enroulée à travers l'ouverture centrale 4 du couvercle 2, cette introduction étant facilitée par la forme conique de sa partie inférieure. Puis le bouchon 21 est fixé dans ladite ouverture 4 du couvercle 2. L'appareil selon l' invention est alors prêt à fonctionner, pour autant bien entendu que le branchement du dispositif de régulation du vide sur l'installation de création de vide à disposition (non montré) ait été effectué. Le sang souillé provenant par exemple d'une sonde utilisée par le chirurgien est donc introduit par aspiration dans la première chambre 13 de la poche 10 par l'intermédiaire du tuyau 15 puis, toujours sous l'effet de l'aspiration par la mise sous vide de l'appareil, le sang passe à travers la paroi filtre-grille 12 jusque dans la seconde chambre 14, depuis laquelle le sang peut être pompé hors de la poche 10 par l'entremise du tuyau 16. La bande hydrophobe 17 que présente cette seconde chambre 14 a une triple utilité : elle sert tout d'abord à la mise sous vide de la poche 10, et ensuite de dispositif d'indication de tropplein de cette seconde chambre, l'aspiration étant en effet interrompue si le niveau du sang dans cette chambre dépasse le niveau supérieur de ladite bande 17. Cette bande hydrophobe constitue également une barrière stérile entre le contenu sanguin et l'extérieur de la poche.

Ainsi, la présente invention fournit un appareil de récupération et de filtration du sang per- et post-opératoire qui présente plusieurs avantages par rapport aux appareils connus :
- Le fait de disposer d'une partie permanente (non jetable) qui comporte tous les branchements sur le système de création du vide permet d'éviter à l'utilisateur des manipulations souvent difficiles et délicates; en effet, le récipient 1 avec son couvercle 2 pouvant être à demeure reliés au système de vide, il suffit d'introduire une poche souple 10 comme indiqué précédemment et de fixer le bouchon 21 hermétiquement dans l'ouverture centrale 4 pour que cette poche soit elle-même mise sous la même pression réduite que le récipient 1, grâce à la présence de la bande hydrophobe 17 mettant en contact l'intérieur du récipient avec l'intérieur de ladite poche, et que le sang puisse être aspiré par l'entremise du tuyau 15, lequel aura été préalablement relié à un autre tuyau amenant le sang du patient. Le branchement de la poche de filtration au système de vide est donc pratiquement automatique.
- Comme expliqué précédemment également, la bande hydrophobe 17 fait office de système de contrôle du trop-plein de la chambre 14 contenant le sang filtré.
- De par sa conception, l'appareil selon l'invention offre une bien meilleure étanchéité, et peut donc être utilisée également pour la collection du sang post-opératoire, plus particlièrement dans des conditions où le système de mise sous vide doit être débranché, par exemple lors du transport du patient, le récipient 1 étant hermétiquement fermé et maintenant le vide pendant un temps suffisamment long.
- Contrairement à d'autres appareils connus dont le volume maximum possible est inférieur à 2 lt, l'appareil selon l'invention peut être réalisé de manière à recevoir des volumes de sang plus importants, par exemple jusqu'à environ 5 lt, ceci notamment grâce au fait qu'il comporte un couvercle rigide, par exemple en métal, qui peut donc présenter un grand diamètre sans risques d'implosion.
- Enfin, la partie jetable de l'appareil selon l'invention, à savoir la poche souple de filtration, est non seulement facile à mettre en oeuvre, mais encore d'une fabrication relativement simple et surtout peu coûteuse.

## Revendications

1. Appareil de récupération et de filtration du sang comportant une enceinte rigide fermée hermétiquement ainsi qu'une poche souple jetable de filtration (10) disposée en position de service à l'intérieur de ladite enceinte, ladite enceinte rigide étant formée par un récipient (1) et un couvercle amovible (2), et ladite poche présentant deux chambres internes séparées l'une de l'autre par une paroi filtrante (12), une première chambre (13) étant reliée à l'extérieur par un tuyau d'entrée (15) alimenté en sang à traiter et une seconde chambre (14) étant reliée à l'extérieur par un tuyau de sortie (16) du sang filtré, caractérisé par le fait que ce couvercle est muni d'un dispositif réglable de branchement (6) à une ligne de mise sous pression réduite et présente une ouverture traversante (4), et par le fait que la paroi filtrante (12) est munie d'une ouverture fermée par un élément (17) en un matériau perméable à l'air et imperméable aux liquides, les deux tuyaux (15,16) respectivement d'entrée et de sortie passant de manière étanche à travers un bouchon (21) fermant en position de service l'ouverture traversante (4) du couvercle (2).

2. Appareil selon la revendication 1, caractérisé par le fait qu'il comporte des joints d'étanchéité (3,22) entre le couvercle (2) et le récipient (1) respectivement entre le bouchon (21) solidaire de la poche souple jetable (10) et l'ouverture traversante (4) du couvercle (2), et par le fait que ledit dispositif réglable de branchement à une ligne de mise sous vide comporte un organe de réglage (6) de la pression et un manomètre (7).

3. Poche souple jetable pour la récupération et la filtration du sang utilisable dans l'appareil selon la revendication 1 et comportant deux chambres internes (13,14) séparées l'une de l'autre par une paroi filtrante (12), une première chambre (13) étant reliée à l'extérieur par un tuyau d'entrée (15) alimenté en sang à traiter et une seconde chambre (14) étant reliée à l'extérieur par un tuyau de sortie (16) du sang filtre, caractérisée par le fait que la paroi est munie d'une ouverture fermée par un élément (17) en un matériau perméable à l'air et imperméable aux liquides, les deux tuyaux (15,16) respectivement d'entrée et de sortie passant de manière étanche à travers un bouchon (21) destiné à fermer en position de service l'ouverture traversante (4) du couvercle (2) de l'appareil.

4. Poche selon la revendication 3, caractérisée par le fait qu'elle est formée par deux parois en matière plastique superposées et d'une grille de filtration (12) également en matière plastique fixée entre ces deux parois par soudure des rebords périphériques (11) de celles-ci.

5. Poche selon la revendication 4, caractérisée par le fait que la paroi de ladite seconde chambre (14) présente une ouverture rectangulaire horizontale dans sa portion supérieure, et par le fait que cette ouverture est fermée par une bande en un matériau hydrophobe (17).

6. Poche selon la revendication 5, caractérisée par le fait que ladite bande hydrophobe (17) est munie au moins d'un côté de rabats mobiles (19,19') en matière plastique.

## Claims

1. Blood collecting and filtering apparatus comprising a tightly closed rigid enclosure as well as a filtration disposable flexible bag (10) disposed in service position inside of said enclosure, said rigid enclosure being formed of a container (1) and a removable cover (2), and said bag having two internal rooms separated the one from the other by a filtering wall (12), a first room (13) being connected to the outside by an inlet pipe (15) which is fed in blood to be treated, and a second room (14) being connected to the outside by an outlet pipe (16) for the filtered blood, characterized by the fact that this cover is provided with an adjustable device (6) for connecting to a reduced pressure line and presenting a traversing opening (4), and by the fact that the fittering wall (12) is provided with an opening which is closed by an element (17) made of an air permeable and liquid tight material, both respectively inlet and outlet pipes (15,16) passing in a tight manner through a plug (21) closing in service position the traversing opening (4) of the cover (2).

2. Apparatus according to claim 1, characterized by the fact that it comprises tightness joints (3,22) between the cover (2) and the container (1) respectively between the plug (21) bound to the disposable flexible bag (10) and the traversing opening (4) of the cover (2), and by the fact that said adjusting device of the connection to a vacuum system comprises an adjustment member (6) of the pressure and a manometer (7).

3. Disposable flexible bag for the collection and the filtration of blood usable with the apparatus according to claim 1 and comprising two internal rooms (13,14) separated the one from the other by a filtering wall (12), a first room (13) being connected to the outside by an inlet pipe (15) which is fed in blood to be treated, and a second room (14) being connected to the outside by an outlet pipe (16) of the filtered blood, characterized by the fact that the wall is provided with an opening closed by an element (17) made of an air permable and liquid tight material, both respectively inlet and outlet pipes (15,16) passing in a tight manner through a plug (21), intended to close in service position the traversing opening (4) of the cover (2) of the apparatus.

4. Bag according to claim 3, characterized by the fact that it is constitued by two superimposed walls of plastic material and of a filtration netting (12) also of plastic material which is fixed between this two walls by welding of the peripheral edges (11) thereof.

5. Bag according to claim 4, characterized by the fact that the wall of said second room (14) has a horizontal rectuangular opening in its upper portion, and by the fact that this opening is closed by a band (17) of a hydrophobic material.

6. Bag according to claim 5, characterized by the fact that said hydrophobic band (17) is provided at least on one side of movable flaps (19,19') of plastic material.

## Patentansprüche

1. Gerät zur Blutrückgewinnung und -filtration mit einem hermetisch verschlossenen, starren Raum sowie einer schmiegsamen Einweg-Filtertasche (10), die ihre Arbeitslage im Inneren des benannten Raumes hat, wobei der benannte starre Raum von einem Behälter (1) und einem abnehmbaren Deckel (2) gebildet wird und die benannte Tasche zwei Innenkammern aufweist, die voneinander durch eine filtrierende Wandung (12) getrennt sind, eine erste Kammer (13) mit der Aussenwelt durch einen Eintrittsschlauch (15) verbunden ist, der mit dem zu behandelnden Blut beschickt wird, und eine zweite Kammer (14) mit der Aussenwelt durch einen Austrittsschlauch (16) für das filtrierte Blut verbunden ist, dadurch gekennzeichnet, dass dieser Deckel mit einer einstellbaren Vorrichtung (6) zum Anschluss an eine Leitung für das Anlegen von vermindertem Druck versehen ist und eine durchgehende Öffnung (4) aufweist und dass die filtrierende Wandung (12) mit einer Öffnung versehen ist, die durch ein Element (17) aus luftdurchlässigem, aber flüssigkeitsundurchlässigem Material verschlossen ist, wobei die beiden Schläuche (15, 16) für den Eintritt bzw. Austritt in hermetisch abgedichteter Weise einen Stöpsel (21) passieren, der in der Arbeitslage die durchgehende Öffnung (4) des Deckels (2) verschliesst.

2. Gerät gemäss Anspruch 1, dadurch gekennzeichnet, dass es Dichtungen (3, 22) zwischen dem Deckel (2) und Behälter (1) bzw. zwischen dem fest mit der schmiegsamen Einwegtasche (10) verbundenen Stöpsel (21) und der durchgehenden Öffnung (4) des Deckels (2) aufweist und dass die benannte einstellbare Vorrichtung zum Anschluss an eine Evakuierungsleitung ein Druckregulierorgan (6) und ein Manometer (7) umfasst.

3. Schmiegsame Einwegtasche für die Blutrückgewinnung und -filtration, die in dem Gerät gemäss Anspruch 1 einsetzbar ist und zwei Innenkammern (13, 14) aufweist, die voneinander durch eine filtrierende Wandung (12) getrennt sind, wobei eine erste Kammer (13) mit der Aussenwelt durch einen Eintrittsschlauch (15) verbunden ist, der mit dem zu behandelnden Blut beschickt wild, und eine zweite Kammer (14) mit der Aussenwelt durch einen Austrittsschlauch (16) für das filtrierte Blut verbunden ist, dadurch gekennzeichnet, dass die Wandung mit einer Öffnung versehen ist, die durch ein Element (17) aus luftdurchlässigem, aber flüssigkeitsundurchlässigem Material verschlossen ist, wobei die beiden Schläuche (15, 16) für den Eintritt bzw. Austritt in hermetisch abgedichteter Weise einen Stöpsel (21) passieren, der dazu bestimmt ist, in der Arbeitslage die durchgehende Öffnung (4) des Gerätedeckels (2) zu verschliessen.

4. Tasche gemäss Anspruch 3, dadurch gekennzeichnet, dass sie aus zwei einander überlagerten Wandungen aus Kunststoff und aus einem gleichfalls aus Kunststoff bestehenden Filternetz (12) gebildet wird, wobei das Filternetz zwischen den beiden Wandungen durch Schweissen entlang der Aussenränder (11) dieser Wandungen eingespannt ist.

5. Tasche gemäss Anspruch 4, dadurch gekennzeichnet, dass die Wandung der benannten zweiten Kammer (14) in ihrer oberen Partie eine waagerechte, rechteckige Öffnung aufweist und dass diese Öffnung durch einen Streifen aus hydrophobem Material (17) verschlossen ist.

6. Tasche gemäss Anspruch 5, dadurch gekennzeichnet, dass der benannte hydrophobe Streifen (17) zumindest einseitig mit beweglichen Kunststoffklappen (19, 19') versehen ist.
